**Europäisches Patentamt**

**European Patent Office**

**Office européen des brevets**

(19)

(11) Numéro de publication : **0 107 994**
**B1**

(12) # FASCICULE DE BREVET EUROPÉEN

(45) Date de publication du fascicule du brevet :
**15.04.87**

(51) Int. Cl.⁴ : **A 61 B 5/10**

(21) Numéro de dépôt : **83401982.0**

(22) Date de dépôt : **11.10.83**

(54) **Procédé pour la détermination de l'absorption percutanée d'un produit chez les mammifères.**

(30) Priorité : **22.10.82 FR 8217737**

(43) Date de publication de la demande :
**09.05.84 Bulletin 84/19**

(45) Mention de la délivrance du brevet :
**15.04.87 Bulletin 87/16**

(84) Etats contractants désignés :
**DE FR GB**

(56) Documents cités :
**CHEMICAL ABSTRACTS, vol. 51, 1957, abrégé no. 185c-d, Columbus, Ohio, US. F. D. MALKINSON et al.: "Percutaneous absorption of cortisone-4-C14 through normal human skin", & J. INVEST. DERMATOL. 28, 211-16, 1957**
**CHEMCIAL ABSTRACTS, vol. 53, 1959, abrégé no. 20528g-h, Columbus, Ohio, US. F. D. MALKINSON: "The percutaneous absorption of carbon-14-labeled steroids by use of the gas-flow cell" & J. INVEST. DERMATOL. 31, 19-28, 1958**
**G. H. BELL et al.: "Textbook of Physiology and Biochemistry", edition 8, 1972, pages 710, 713, 714, Churchill, Livingstone, Edinburgh et Londres, GB**

(73) Titulaire : **L'OREAL**
**14, Rue Royale**
**F-75008 Paris (FR)**

(72) Inventeur : **Rougier, André**
**10, avenue du Général Leclerc**
**F-77230 Dammartin en Goele (FR)**
Inventeur : **Dupuis, Didier**
**44, allée des Coteaux**
**F-93340 Le Raincy (FR)**
Inventeur : **Lotte, Claire**
**43 Bis, Quai Louis Ferber**
**F-94360 Brie S/Marne (FR)**
Inventeur : **Roguet, Roland**
**11, avenue Eugénie**
**F-92700 Colombes (FR)**

(74) Mandataire : **Peuscet, Jacques**
**Cabinet Peuscet 68, rue d'Hauteville**
**F-75010 Paris (FR)**

## Description

L'invention concerne la détermination de l'absorption percutanée d'un produit chez les mammifères.

On sait qu'il est particulièrement important de déterminer dans quelle mesure un produit mis en contact avec la peau d'un mammifère, notamment avec la peau de l'homme, est susceptible de passer la barrière cutanée et de se retrouver dans l'organisme. La détermination de l'absorption percutanée est notamment très utile en cosmétique ou dans le domaine médical pour pouvoir définir des compositions cosmétiques ou médicinales permettant de limiter ou d'augmenter selon le cas la pénétration des produits utilisés à l'intérieur de l'organisme.

Dans l'état de la technique, on a indiqué que le stratum corneum constituait la barrière principale s'opposant au passage des molécules à l'intérieur de l'organisme et l'on a défini que le stratum corneum jouait un rôle de réservoir pour les molécules appliquées sur la peau (voir notamment MALKINSON FD et FERGUSON EH, J. Invest. Dermatol. 25, 281-283, 1955). On a également constaté que les mesures de la pénétration *in vivo* sont fonction des techniques utilisées, de l'espèce animale étudiée, de l'emplacement anatomique soumis à l'application, de la concentration et de la nature des produits testés et de la durée du temps d'application. Jusqu'à ce jour, pour déterminer la quantité de produit ayant pénétré dans un organisme de mammifère après une application d'un produit, il était nécessaire de sacrifier le mammifère traité ; il en résulte qu'il était impossible d'obtenir des résultats précis concernant l'homme, ce qui constituait un obstacle pour l'amélioration des caractéristiques d'innocuité des compositions cosmétiques.

La présente invention a pour but de proposer un procédé pour la détermination de l'absorption percutanée d'un produit chez les mammifères. Selon l'invention, on a constaté que si l'on déterminait la quantité de produit qui, après l'application sur la peau, reste stockée dans le stratum corneum, on pouvait facilement en déduire pour les conditions d'application données et pour un mammifère donné, la quantité totale de produit qui sera introduite dans l'organisme. Selon l'invention, on a constaté que le procédé de détermination était utilisable quel que soit le produit testé et qu'il suffit donc d'en définir les paramètres au moyen de produits ayant une totale innocuité pour pouvoir utiliser le procédé afin de déterminer l'absorption percutanée pour des produits éventuellement moins inoffensifs.

La présente invention a, en conséquence, pour objet, un procédé pour la détermination de la quantité y d'un produit qui a pénétré dans le corps d'un mammifère après que la peau dudit mammifère ait été mise en contact avec ledit produit pendant un temps $t_0$, procédé suivant lequel, après la mise en contact peau/produit et après lavage de la zone de la peau traitée, on prélève sur la peau traitée une zone de stratum corneum, on détermine la quantité X de produit contenu dans le stratum corneum prélevé, et on déduit la quantité y de la quantité X par une relation $Y = aX - b$ avec $y = 0$ si Y est négatif ou nul et $y = Y$ si Y est positif, formule dans laquelle a et b sont des constantes positives prédéterminées et indépendantes du produit appliqué sur la peau.

Dans un mode préféré de mise en œuvre du procédé selon l'invention, on effectue le prélèvement de stratum corneum par arrachages (ou strippings) successifs des couches de stratum corneum ; on effectue, de préférence, six à quinze strippings successifs, chaque stripping étant réalisé au moyen d'un ruban adhésif que l'on applique sur la peau puis que l'on arrache ; on effectue le prélèvement de stratum corneum juste après la fin de la mise en contact du produit avec la peau ; on met en contact le produit testé avec la peau sous forme liquide, notamment en solution ou en formulation.

Pour mieux faire comprendre l'objet de l'invention, on va décrire ci-après, des expériences réalisées sur le rat Hairless et sur l'homme pour définir des relations $Y = aX - b$ utilisables pour ces mammifères.

A. Expériences réalisées sur des rats Hairless

On a testé la pénétration percutanée de dix molécules radiomarquées à savoir l'acide benzoïque (cycle $^{14}C$) à deux concentrations (respectivement produit A et B), l'acide acétyl (carboxyle $^{14}C$) salicylique (produit C), la déhydro (1,2,6,7 $^3H$) épiandrostérone (produit D), le salicylate (carboxyle $^{14}C$) de sodium (produit E), la testostérone (4 $^{14}C$) (produit F), la caféine (8 $^3H$) (produit G), la thiourée ($^{14}C$) (produit H), le D-mannitol (1 $^{14}C$) (produit I), l'hydrocortisone (1,2,6,7 $^3H$) (produit J), la dexaméthasone (1,2 $^3H$) (produit K).

L'activité mise en œuvre pour chaque produit et le solvant utilisé sont indiqués dans le tableau I. Pour chaque produit, on utilise un lot de douze rats Sprague-Dawley Hairless femelles âgés de douze semaines pesant $240 \pm 10$ g. Les produits testés sont appliqués sur 1 cm² de la peau du dos des animaux préalablement anesthésiés par administration intrapéritonéale de gamma butyrolactone à la dose de 0,5 ml/kg. La surface d'application est délimitée par une cellule circulaire ouverte de 1 cm² maintenue en place par une colle siliconée. Les produits sont déposés sur la peau à des doses et dans des solvants définis dans le tableau I. La durée de l'application a été limitée à 30 minutes. L'excès de produit se trouvant sur la zone traitée est ensuite éliminé par une série de deux lavages utilisant le solvant qui a servi de véhicule au produit (voir tableau I) et de deux rinçages à l'eau distillée ; on sèche ensuite légèrement la zone avec un coton.

Le lot de douze animaux est alors partagé en deux groupes égaux. Le stratum corneum de la zone d'application de chaque animal du premier groupe est prélevé par une série de six strippings pratiquée avec du ruban adhésif vendu par la société « 3M » ; on sait que de la sorte, on enlève la majeure partie du stratum corneum, la couche cornée de la peau du rat Hairless ayant une épaisseur de 10 à 15 μ et étant constituée de 7 à 8 assises cellulaires. La radioactivité portée par chaque ruban est mesurée après digestion complète du matériau kératinique dans un produit « Soluène 350 (Packard) » puis addition du produit « Dimilume 30 (Packard) » ; on compte en scintillation liquide (Packard 460 C) ; on obtient ainsi les quantités de produit contenues dans le stratum corneum.

Les animaux du deuxième groupe, munis de collerettes afin d'éviter tout léchage, sont placés individuellement pendant 4 jours dans des cages à métabolisme. Pendant cette période, la nourriture (Extra-labo) et l'eau de ville sont fournies «ad libitum». Les urines et les fèces sont collectées quotidiennement ; les fèces sont lyophilisées et homogénéisées ; après combustion des échantillons à l'« Oxidizer 306 (Packard) », on compte globalement la radio-activité en scintillation liquide. Après quatre jours, les animaux sont sacrifiés. Une série de six strippings est pratiquée sur la zone d'application afin de déterminer la quantité de produit non pénétrée 96 heures après le début de l'expérience. Le reste de la peau de la zone traitée (épiderme + derme) est prélevé et compté en scintillation liquide après digestion dans le produit « Soluène 350 ». Les animaux sont lyophilisés, homogénéisés et les échantillons sont comptés en scintillation liquide après combustion. Pour les molécules marquées au tritium, on prend soin de compter la radio-activité présente dans l'eau de lyophilisation et d'en tenir compte dans l'établissement du bilan final. La quantité totale de produit pénétrée en 96 heures est donc déterminée par addition des quantités retrouvées dans les excrétats (urine et fèces), l'épiderme et le derme du site d'application et dans le corps de l'animal. Ces quantités sont données dans le tableau II. Sur ce tableau ainsi que sur le tableau suivant, on a fait figurer le nombre n (qui est ici égal à 6) de sujets traités, ce qui signifie que chacun des résultats reportés dans ces tableaux représente la moyenne arithmétique des n résultats donnés par l'expérience.

Le tableau III indique les quantités de produit présentes dans le stratum corneum à la fin du temps d'application de 30 minutes et les quantités totales pénétrées en 96 heures. On constate que les quantités totales y peuvent être obtenues à partir des quantités X présentes dans le stratum corneum après l'application grâce à la relation $y = 1,644 \, X - 0,536$. Il est clair que y étant toujours positif, la relation susmentionnée ne s'applique qu'à compter du moment où l'expression $(1,644 - 0,536)$ est positive, y restant nul lorsque cette expression est négative, c'est-à-dire lorsque la quantité de produit appliquée est suffisamment faible. Le traitement statistique des résultats montre que les variables y et X sont corrélées avec un coefficient de 0,998.

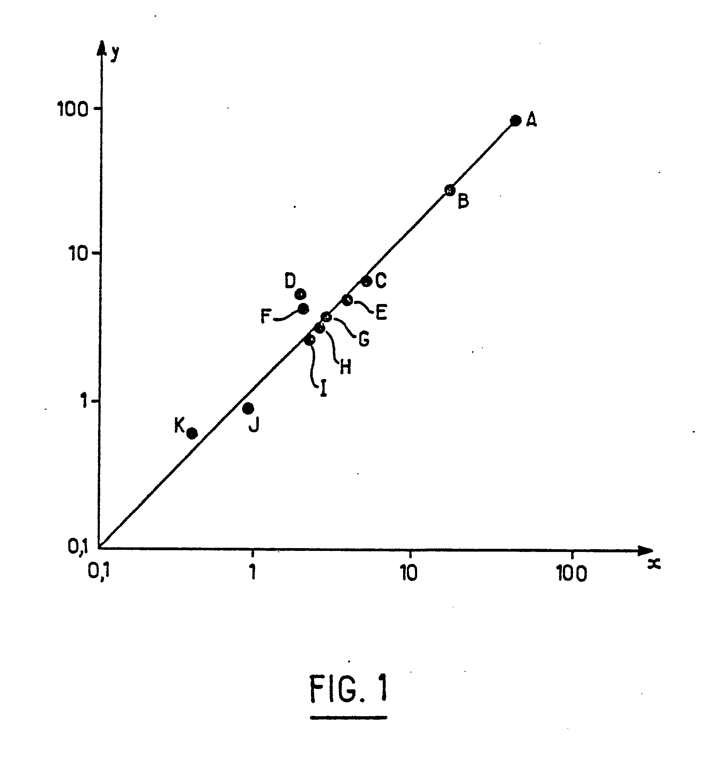

Sur la figure 1, on a représenté, en coordonnées logarithmiques, les résultats fournis dans le tableau III, les points du graphique étant identifiés par les lettres qui désignent les produits correspondants. On a porté en ordonnées la quantité y de produit ayant pénétré dans l'organisme en 96 heures exprimée en nano-moles par $cm^2$ et en abscisses la quantité x de produit présente dans le stratum corneum après 30 minutes d'application exprimée en nano-moles par $cm^2$ également.

B. Expériences réalisées en parallèle sur des rats Hairless et sur des hommes

Les expériences qui viennent d'être décrites, réalisées sur dix molécules radiomarquées de propriétés physicochimiques très différentes, ont donc montré la relation linéaire existant, pour une modalité d'application donnée, entre les quantités de substance présentes dans le réservoir du stratum corneum en fin d'application, c'est-à-dire après 30 minutes, et les quantités totales pénétrées en 4 jours, ce qui permet une approche prédictive de l'absorption percutanée des molécules chez cette espèce. On a pu également remarquer que cette relation est, pour une molécule donnée, indépendante de la dose administrée. Il restait donc à vérifier que le même type de phénomène existe chez d'autres espèces et, en particulier, chez l'homme.

L'étude ci-après établit la relation existant entre le réservoir du stratum corneum et l'absorption percutanée de substances, comparativement chez l'homme et chez le rat.

On a testé la pénétration percutanée de quatre doses, respectivement 125, 250, 500 et 1 000 nano-moles, d'une molécule radiomarquée, à savoir l'acide benzoïque (cycle $^{14}C$). Le choix de l'acide benzoïque a été guidé par l'innocuité de cette molécule, son excellente absorption percutanée et sa rapide et quasi-totale élimination par voie urinaire. L'acide benzoïque utilisé a été fourni par « The Radio-Chemical Center Amersham ».

L'activité spécifique mise en œuvre pour ce produit est égale à 0,1 micro-Curie/nano-mole et les doses de ce produit ont été appliquées en solution dans l'éthylène-glycol auquel on a ajouté 10 % d'alkylphényl polyéthylèneglycol (agent tensio-actif non ionique vendu sous le nom commercial « Triton X100 ») afin d'en augmenter la mouillabilité. Chaque dose à appliquer est contenue dans 20 μl du mélange précité. Pour chaque dose de produit, on utilise un lot de douze rats Sprague-Dawley Hairless femelles âgés de douze semaines pesant $240 \pm 10$ g et un groupe de cinq à sept patients mâles, de race caucasienne, âgé de $25 \pm 0,8$ ans et pesant $74 \pm 1,6$ kg.

Chez le rat, les doses du produit testé sont appliquées sur 1 $cm^2$ de la peau du dos de ces animaux préalablement anesthésiés par administration intrapéritonéale de gamma-butyrolactone à la dose de 0,5 ml par kg. Chez l'homme, l'administration des différentes doses du produit a été pratiquée sur la face externe du bras, sur 1 cm² de peau.

Tant chez l'homme que chez le rat, la surface d'application est délimitée par une cellule circulaire ouverte de 1 cm² maintenue en place par une colle siliconée (Tecsil). Les doses de produit, dans le solvant précité, sont déposées sur la peau, la durée de l'application étant limitée à 30 minutes. L'excès de produit se trouvant dans la zone traitée est éliminée rapidement par une série de deux lavages avec, à chaque fois, 300 microlitres d'un mélange éthanol/eau dans un rapport volumique 95/5, et de deux rinçages avec, à chaque fois, 300 microlitres d'eau distillée ; on sèche ensuite légèrement la zone avec un coton.

A la fin de la procédure d'application, chaque lot de douze animaux est alors partagé en deux groupes égaux. Les animaux des premiers groupes sont utilisés pour déterminer les teneurs en acide benzoïque présentes dans le réservoir du stratum corneum à la fin du temps d'application. La procédure utilisée pour cette détermination est identique à celle décrite précédemment.

Les animaux des deuxièmes groupes sont utilisés pour la mesure des quantités totales d'acide benzoïque pénétrées en 96 heures, obtenues par sommation des quantités retrouvées dans les excrétats (urine + fècès), dans l'épiderme et le derme de la zone traitée et dans le corps des animaux. La procédure utilisée pour cette détermination est identique à celle décrite précédemment.

Chez l'homme, chaque groupe a d'abord reçu une dose d'acide benzoïque, l'application ayant lieu sur 1 cm² de la face externe du bras droit suivant la modalité d'application indiquée plus haut.

L'absorption percutanée totale en 4 jours de l'acide benzoïque chez l'homme a été déduite du résultat de la mesure en scintillation liquide des urines émises pendant les 48 heures suivant l'application. L'étude des cinétiques d'élimination urinaire de l'acide benzoïque a montré que l'excrétion par cette voie est extrêmement rapide ; on a ainsi pu constater que, chez le rat, les quantités d'acide benzoïque trouvées dans les urines de 48 heures représentent 80 % des quantités pénétrées en 4 jours. Ce taux de recouvrement est en accord avec celui obtenu lors de l'administration par voie intraveineuse de cette molécule. De plus, il a été démontré que l'acide benzoïque administré chez l'homme ou chez le rat par la voie orale était totalement retrouvé dans les urines de 24 heures. L'ensemble de ces données a donc permis de calculer chez l'homme la quantité totale d'acide benzoïque pénétrée en 4 jours à partir des quantités excrétées par voie urinaire en 48 heures, considérant que celles-ci représentent environ 80 % de la quantité totale absorbée.

Afin de mesurer l'effet réservoir du stratum corneum, chaque sujet reçoit 5 jours après la première application, sur la face externe du bras gauche, la même dose d'acide benzoïque que lors de la première administration sur le bras droit.

En fin de procédure d'application, 15 strippings sont pratiqués sur la zone traitée, le principe de la mesure de la radioactivité présente dans chacun des strippings étant le même que celui précédemment décrit.

Le tableau IV montre :

1° Chez le rat :

— la distribution de l'acide benzoïque dans les voies d'excrétion urinaire et fécale ainsi que sa rétention corporelle ;
— les taux de pénétration des doses d'acide benzoïque 96 heures après leur administration ; et
— les quantités d'acide benzoïque présentes dans le stratum corneum à la fin du temps d'application, c'est-à-dire après 30 minutes.

2° Chez l'homme :

— les quantités d'acide benzoïque excrétées par voie urinaire dans les 48 heures suivant l'application ;
— les taux de pénétration en 96 heures des différentes doses d'acide benzoïque, calculés à partir des quantités excrétées par la voie urinaire en 48 heures ; et
— les quantités d'acide benzoïque présentes dans la couche cornée à la fin de la période d'application, c'est-à-dire après 30 minutes.

Sur ce tableau, on a également fait figurer le nombre n de sujets traités, chacun des résultats reportés dans ce tableau représentant par conséquent la moyenne arithmétique des n résultats obtenus.

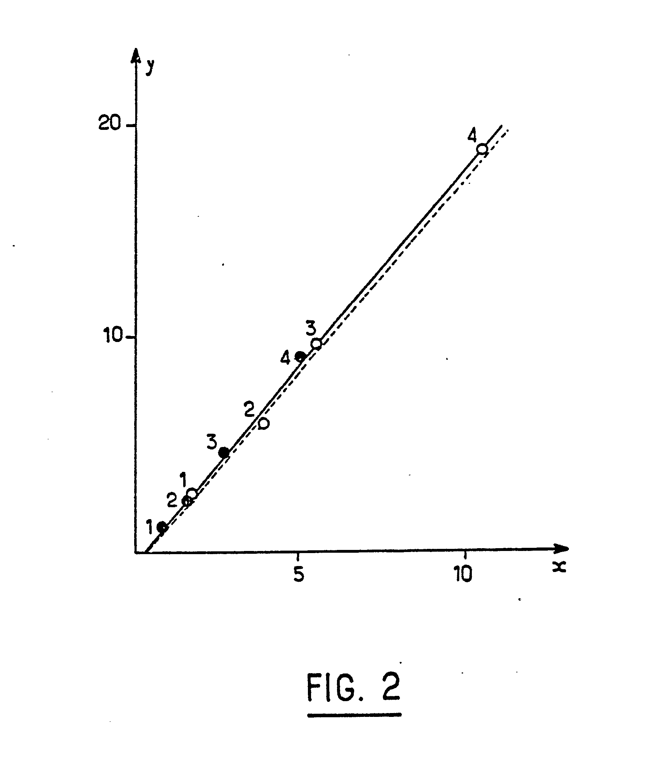

Sur la figure 2, on a représenté, en coordonnées linéaires, les résultats fournis par le tableau IV, les points du graphique étant identifiés par les chiffres 1 à 4 qui correspondent à l'application d'une dose respectivement de 125, 250, 500 et 1 000 nano-moles d'acide benzoïque par cm² de peau, les points matérialisés par des disques pleins noirs représentant les résultats obtenus pour l'homme et ceux matérialisés par de simples cercles étant ceux obtenus pour le rat. On a porté, en ordonnées, la quantité y de produit ayant pénétré dans l'organisme en 96 heures exprimée en nano-moles par cm² et, en abscisses, la quantité x de produit présente dans le stratum corneum après 30 minutes d'application exprimée en nano-moles par cm² également.

Il est remarquable de voir que les points obtenus pour les deux espèces étudiées ici peuvent être considérés, pour la modalité particulière d'application, comme appartenant à la même droite, du type

$y = ax - b$, où $a = 1,83$ et $b = 0,52$. Le traitement statistique des résultats montre que les variables y et X sont corrélées avec un coefficient de 0,998. On peut remarquer également que la droite représentée en traits pleins sur la figure 2 est pratiquement identique à la droite établie lors de l'étude décrite au paragraphe A, cette dernière droite étant représentée en traits pointillés sur la figure 2. Compte tenu des incertitudes des mesures, dues notamment aux différents lots d'animaux utilisés, on peut considérer que ces deux droites ne sont pas « statistiquement » distinctes et que, par conséquent, les valeurs de a et b déterminées dans l'un et l'autre cas sont les mêmes.

On constate qu'il suffit, pour pouvoir appliquer le procédé selon l'invention, de déterminer la droite correspondant à un mammifère donné et à une modalité d'application donnée, pour que la simple connaissance de la quantité de produits retenus dans le stratum corneum en fin d'application permette de savoir quelle est l'absorption percutanée totale dans l'organisme pour le produit considéré.

Il est bien entendu que les modes de mise en œuvre ci-dessus décrits ne sont aucunement limitatifs et pourront donner lieu à toute modification désirable, sans que l'on s'écarte pour cela du cadre de l'invention, qui est définie dans les revendications jointes.

(Voir Tableaux pages suivantes)

Tableau I

| MOLECULES | P.M. | Véhicule de la molécule appliquée : 20 μl d'un mélange éthanol/eau | Quantité appliquée | Activité mise en oeuvre | Solution de rinçage éthanol/eau |
|---|---|---|---|---|---|
| | | Vol. éthanol/vol. eau | $n-moles/cm^2$ | micro-Curies | vol. éthanol/vol. eau |
| ACIDE BENZOÏQUE | 122 | 95/5 <br> 95/5 | 450 <br> 200 | 13,0 <br> 13,0 | 95/5 <br> 95/5 |
| ACIDE ACETYL-SALICYLIQUE | 180 | 95/5 | 200 | 4,0 | 95/5 |
| DEHYDROEPI-ANDROSTERONE | 288 | 95/5 | 200 | 83,0 | 95/5 |
| SALICYLATE DE SODIUM | 160 | 50/50 | 200 | 4,0 | 50/50 |
| TESTOSTERONE | 290 | 95/5 | 200 | 12,5 | 95/5 |
| CAFEINE | 194 | 95/5 | 200 | 19,5 | 95/5 |
| THIOUREE | 76 | 95/5 | 200 | 12,5 | 95/5 |
| MANNITOL | 182 | 25/75 | 200 | 1,5 | 25/75 |
| HYDROCORTISONE | 369 | 95/5 | 200 | 10,5 | 95/5 |
| DEXAMETHASONE | 392 | 95/5 | 200 | 99,5 | 95/5 |

0 107 994

Tableau II

| MOLECULES | URINES | FECES | EPIDERME + DERME zone traitée | CORPS DES ANIMAUX | TOTAL PENETRE EN 96 h | |
|---|---|---|---|---|---|---|
| | | | | | $n\text{-moles/cm}^2$ | % DE LA ZONE APPLIQUEE |
| ACIDE BENZOÏQUE 450 nanomoles/cm$^2$ | 71,2 (2,90)* | 6,25 (1,65) | 1,70 (0,40) | 0,16 (0,02) | 79,30 (3,60) | 17,60 (0,80) |
| 200 nanomoles/cm$^2$ | 24,80 (0,50) | 1,32 (0,30) | 0,50 (0,05) | 0,01 (0,004) | 26,60 (0,65) | 13,30 (0,32) |
| ACIDE ACETYL-SALICYLIQUE | 5,40 (0,70) | 0,43 (0,15) | 0,85 (0,20) | 0 | 6,70 (0,70) | 3,35 (0,35) |
| DEHYDROEPI-ANDROSTERONE | 1,67 (0,15) | 3,00 (0,30) | 0,19 (0,03) | 0,43 (0,08) | 5,28 (0,20) | 2,65 (0,10) |
| SALICYLATE DE SODIUM | 3,50 (0,50) | 0,48 (0,10) | 1,00 (0,17) | 0 | 4,98 (0,70) | 2,50 (0,35) |
| TESTOSTERONE | 1,72 (0,43) | 2,05 (0,60) | 0,24 (0,05) | 0,33 (0,15) | 4,32 (0,45) | 2,16 (0,23) |
| CAFEINE | 2,52 (0,25) | 0,42 (0,07) | 0,15 (0,01) | 0,65 (0,06) | 3,73 (0,30) | 1,86 (0,15) |
| THIOUREE | 2,10 (0,40) | 0,76 (0,04) | 0,20 (0,02) | 0,20 (0,04) | 3,23 (0,50) | 1,61 (0,25) |
| MANNITOL | 1,60 (0,20) | 0,42 (0,15) | 0,30 (0,10) | 0,30 (0,10) | 2,63 (0,17) | 1,32 (0,08) |
| HYDROCORTISONE | 0,46 (0,07) | 0,33 (0,07) | 0,04 (0,01) | 0,03 (0,01) | 0,86 (0,08) | 0,43 (0,04) |
| DEXAMETHASONE | 0,16 (0,02) | 0,30 (0,05) | 0,10 (0,02) | 0,04 (0,01) | 0,60 (0,06) | 0,30 (0,03) |

* Ecart-type (n = 6)

0 107 994

**Tableau III**

| DESIGNATION DES PRODUITS | PRODUITS TESTES | NANO-MOLES DE PRODUITS | |
|---|---|---|---|
| | | PENETREES EN 96 HEURES | PRESENTES DANS LE STRATUM CORNEUM DE LA ZONE TRAITEE APRES 30 MINUTES D'APPLICATION |
| A | ACIDE BENZOÏQUE 450 n-moles/$cm^2$ | 79,30 + (3,60)* | 48,10 (5,10) |
| B | 200 n-moles/$cm^2$ | 26,60 (0,70) | 17,60 (1,50) |
| C | ACIDE ACETYL-SALICYLIQUE | 6,70 (0,70) | 5,15 (0,64) |
| D | DEHYDROEPI-ANDROSTERONE | 5,28 (0,20) | 1,95 (0,40) |
| E | SALICYLATE DE SODIUM | 4,98 (0,71) | 3,85 (0,29) |
| F | TESTOSTERONE | 4,32 (0,46) | 2,06 (0,23) |
| G | CAFEINE | 3,73 (0,30) | 2,76 (0,34) |
| H | THIOUREE | 3,23 (0,48) | 2,63 (0,33) |
| I | MANNITOL | 2,63 (0,17) | 2,22 (0,37) |
| J | HYDROCORTISONE | 0,86 (0,08) | 0,93 (0,15) |
| K | DEXAMETHASONE | 0,60 (0,06) | 0,41 (0,02) |

* Ecart-type (n = 6) + nano-moles/$cm^2$

Tableau IV

| ACIDE BENZOÏQUE APPLIQUE (nanomole/cm²) | | QUANTITES TOTALES TROUVEES 4 JOURS APRES ADMINISTRATION TOPIQUE | | | | PENETRATION TOTALE EN 4 JOURS (b) | QUANTITES PRESENTES DANS LE STRATUM CORNEUM DE LA ZONE TRAITEE APRES 30 MINUTES D'APPLICATION |
|---|---|---|---|---|---|---|---|
| | | URINES (a) | FECES | EPIDERME + DERME zone traitée | CORPS DES ANIMAUX | | |
| 125 | rat n = 5 | 2,52 (c) (0,43) (d) | 0,14 (0,03) | 0,05 (0,008) | 0 | 2,71 (0,44) | 1,69 (0,18) |
| | homme n = 5 | 0,92 (0,06) | — | — | — | 1,14 (0,07) | 0,81 (0,04) |
| 250 | rat n = 5 | 5,23 (0,92) | 0,48 (0,15) | 0,15 (0,06) | 0,03 (0,03) | 5,89 (1,06) | 3,88 (0,69) |
| | homme n = 6 | 1,84 (0,22) | — | — | — | 2,30 (0,28) | 1,61 (0,18) |
| 500 | rat n = 5 | 8,24 (0,72) | 1,06 (0,37) | 0,22 (0,05) | 0,05 (0,04) | 9,57 (0,74) | 5,47 (0,64) |
| | homme n = 5 | 3,65 (1,20) | — | — | — | 4,56 (1,49) | 2,64 (0,36) |
| 1000 | rat n = 5 | 16,25 (2,38) | 2,03 (0,27) | 0,40 (0,13) | 0,06 (0,02) | 18,75 (2,57) | 10,46 (0,98) |
| | homme n = 7 | 7,12 (2,10) | — | — | — | 8,90 (2,63) | 5,01 (1,06) |

(a) pour l'homme : quantités trouvées au cours des premières 48 heures
(b) pour l'homme : valeurs corrigées à partir de (a)
(c) exprimé en nano-moles par cm² de zone d'application
(d) écart-type

# 0 107 994

**Revendications**

1. Procédé pour la détermination de la quantité y d'un produit, qui a pénétré dans le corps d'un mammifère après que la peau dudit mammifère a été mise en contact avec ledit produit pendant un temps $t_0$, procédé suivant lequel, après la mise en contact peau/produit et après lavage de la zone d'épiderme traitée, on prélève sur la peau traitée, une zone de stratum corneum, on détermine la quantité X de produit contenue dans le stratum corneum prélevé, et on déduit la quantité y de la quantité X par une relation $Y = aX - b$ avec $y = 0$ si Y est négatif ou nul et $y = Y$ si Y est positif, formule dans laquelle a et b sont des constantes positives prédéterminées et indépendantes du produit appliqué sur la peau, toute méthode de diagnostic appliquée au corps humain ou animal au sens de l'article 52 (4) CBE étant exclue.

2. Procédé selon la revendication 1, caractérisé par le fait que l'on effectue le prélèvement de stratum corneum par arrachages (ou strippings) successifs des couches de stratum corneum.

3. Procédé selon la revendication 2, caractérisé par le fait que l'on effectue six à quinze strippings successifs, chaque stripping étant réalisé au moyen d'un ruban adhésif que l'on applique sur la peau puis que l'on arrache.

4. Procédé selon l'une des revendications 1 à 3, caractérisé par le fait que l'on effectue le prélèvement de stratum corneum juste après la fin de la mise en contact du produit avec la peau.

5. Procédé selon l'une des revendications 1 à 4, caractérisé par le fait que l'on met en contact le produit testé avec la peau sous forme liquide.

**Claims**

1. A process for the determination of the quantity of a product which has penetrated into the body of a mammal after the skin of said mammal has been contacted with said product for a time $T_0$, in which process, after the contacting of skin/product and after washing of the epidermal zone treated : there is provided on the treated zone a zone of stratum corneum ; the quantity X of the product contained in the stratum corneum is determined ; and the quantity y is deduced from the quantity X by a relation $Y = aX - b$ with $y = 0$ if Y is negative or zero, and $y = Y$ if Y is positive, a and b being predetermined positive constants in said relation and independent of the product applied to the skin, all diagnostic methods applied to the human body or to the animal body within the ambit of Article 52 (4) EPC being excluded.

2. A process according to claim 1, characterised by the fact that the stratum corneum is provided by successive tearing out or stripping of layers of stratum corneum.

3. A process according to claim 2, characterised by the fact that six to fifteen successive strippings are carried out, each stripping being effected by means of an adhesive tape which is applied to the skin and then torn off.

4. A process according to one of claims 1 to 3, characterised by the fact that the provision of the stratum corneum is carried out just after the end of the contacting of the product with the skin.

5. A process according to one of claims 1 to 4, characterised by the fact that the product to be tested is placed into contact with the skin in liquid form.

**Patentansprüche**

1. Verfahren zur Bestimmung der Menge y eines Produkts, das in den Körper eines Säugetiers eingedrungen ist, nachdem die Haut dieses Säugetiers mit diesem Produkt während eines Zeitraums $t_0$ in Kontakt gebracht worden ist, wobei man, nachdem man die Haut mit dem Produkt in Kontakt gebracht hat und nachdem man die behandelte Epidermiszone gewaschen hat, von der behandelten Haut eine Zone des Stratum Corneum abnimmt, die Menge X des im abgenommenen Stratum Corneum enthaltenen Produkts bestimmt und die Menge y aus der Menge X mit Hilfe einer Gleichung $Y = aX - b$ bestimmt, wobei $y = 0$ ist, wenn Y negativ oder null ist und $y = Y$ ist, wenn Y positiv ist, und wobei in dieser Formel a und b positive, vorbestimmte und von dem auf die Haut aufgetragenen Produkt unabhängige Konstanten sind, mit der Maßgabe, daß jedes am menschlichen oder tierischen Körper vorgenommene Diagnostizierverfahren gemäß Artikel 52 (4) EPÜ ausgenommen ist.

2. Verfahren nach Anspruch 1, dadurch gekennzeichnet, daß man das Stratum Corneum abnimmt, indem man Stratum-Corneum-Schichten nacheinander abreißt oder abzieht (Strippings).

3. Verfahren nach Anspruch 2, dadurch gekennzeichnet, daß man 6 bis 15 aufeinanderfolgende Strippings durchführt, wobei man jedes Stripping mit Hilfe eines Klebebandes vornimmt, das man auf der Haut anbringt und dann abreißt.

4. Verfahren nach einem der Ansprüche 1 bis 3, dadurch gekennzeichnet, daß man das Stratum Corneum abnimmt, kurz nachdem man das Produkt mit der Haut in Kontakt gebracht hat.

5. Verfahren nach einem der Ansprüche 1 bis 4, dadurch gekennzeichnet, daß man das zu testende Produkt in flüssiger Form mit der Haut in Kontakt bringt.

FIG. 1

FIG. 2